# EUROPEAN PATENT APPLICATION

(11) **EP 4 772 172 A1**
(43) Date of publication of application: **08.07.2026**
(21) Application number: 23950137.2
(22) Date of filing: 30.08.2023
(51) Int. Cl.: A61K 9/127, A61K 47/68, A61K 39/44, A61K 31/4745, A61P 35/00, A61P 37/02

(54) **T CELL-DIRECTED IMMUNOLIPOSOME AND USE THEREOF**

(71) Applicant: Highfield Biopharmaceuticals Corporation, HangZhou, Zhejiang 310051 (CN)
(72) Inventor: XU, Yuhong, Hangzhou, Zhejiang 310051 (CN); ZHENG, Anjie, Hangzhou, Zhejiang 310051 (CN); JIN, Shanshan, Hangzhou, Zhejiang 310051 (CN); XIE, Fang, Hangzhou, Zhejiang 310051 (CN); XU, Fengwei, Hangzhou, Zhejiang 310051 (CN); DAI, Yongchao, Hangzhou, Zhejiang 310051 (CN)
(74) Representative: Lavoix
(86) International application number: PCT/CN2023/115857
(87) International publication number: WO 2025/043547

(57) **Abstract**

The present invention relates to the field of uses ofliposome formulations in preparation of tumor drugs, and in particular to a T cell-directed immunoliposome (TRAFsome). A targeting antibody and an immune cell engaging antibody have different valencies and are optimized separately, and a drug encapsulated in the liposome can be an immunomodulator, an anti-cancer drug or a combination thereof.

## Description

### Technical Field

The present disclosure belongs to the technical field of pharmaceutical preparations, liposome drug carriers and antibody targeting, and particularly relates to a T cell redirecting liposome pharmaceutical preparation that acts both with immune cells and target cells.

### Background

Liposome is a drug carrier with a bilayer vesicle-like structure based on phospholipid and/or cholesterol. Liposomes are artificially prepared bilayer vesicles formed by lipid molecules. Since the lipid molecule has a hydrophilic head and a hydrophobic tail, hydrophilic drugs can be contained in the internal aqueous phase, and hydrophobic drugs can be embedded in the lipid bilayer. In addition, liposomes have good biocompatibility, low toxicity and good bio-safety, therefore have attracted widespread attention and been applied in the field of drug delivery systems. In particular, the development of liposome targeting technology has endowed the lipid drug carrier with unique in vivo behavior, enabling it to accumulate at the target site and then interact with the target cell specifically. It is hoped that by optimizing the selection of targets and improving the structure and performance of targeting ligands, clinical efficacy of liposome drugs can be improved, including reducing drug system toxicity and increasing therapeutic index.

The research and development idea of targeting liposome drug delivery system is to design various types of active targeting ligands and drug carriers, targeting to the receptors that are highly expressed or specifically expressed on the surface of tumor cells. The active targeting drugs developed according to the above design idea have more optimized targeting capability at the lesion site than the free drugs delivered without targeting carriers, for example, the drug concentration at the target site is increased, the pharmacodynamics is improved, etc. However, this type of targeting drug delivery system only acts on a specific type of cells, it may not effectively clear the tumor, and may induce the problems such as minimal residual disease and multidrug resistance of the tumor.

In recent years, the understanding of tumor immunology is improved, therefore the mechanism of the limitation of tumor cell only targeting has been elucidated. For example, the differences in molecular markers between different tumor cells within the same tumor in the same patient, the different types of immune cells contributing to the immune suppressive microenvironment, and the interactions within the tumor environment. Aiming at the characteristics of tumor microenvironment, the researchers have expanded the design idea of targeting delivery. For example, drug carriers targeting tumor-related cells can inhibit tumor growth through other mechanisms, such as acting on tumor new blood vessels, T cells, myeloid cells, and tumor stem cells.

Although such targeting delivery technology has improved therapeutic, the design idea still resides within the established framework of single cell type targeting. There are a wide range of dynamic interactions between different cells within the tumor, such as lymphocyte infiltration, macrophage phagocytosis, antigen presentation, and intercellular communication based on cytokines and exosomes. These interactions include immune surveillance and clearance to inhibit tumor growth, as well as the microenvironment that promotes tumor growth. Moreover, blocking or promoting these intercellular interactions has proven to be feasible and effective in clinical practice, such as vaccine therapy based on antigen-specific T cells, antibody therapy that relies on the cytotoxic effect of NK cell, and immune checkpoint inhibitor therapy for blocking tumor from immune escaping. In contrast, single cell type targeting drug delivery systems can only regulate physiological processes by aiming at specific target cells, but cannot modify the intercellular interactions in tumor tissues to achieve effective treatment.

Therefore, in the present disclosure, different antibodies against different cell types are loaded onto a single liposome. The liposome can sequentially or simultaneously bind to different type of cells by means of the action of antibodies, to promote the recognition, communication, activation, pro-apoptosis or clearance of the target cells, etc. In addition, targeting drug delivery for multiple cell types using a single liposome is achieved by the stable lipid carrier structure, which avoids problems such as drug interactions that may occur in combination therapy, and improves the pharmacological effects.

### Summary

The present disclosure provides an immune cell redirecting immuno-liposome, also known as T-cell redirecting antibody fragment-anchored liposome (TRAFsome), a pharmaceutical preparation capable of sequentially or simultaneously binding to multiple different cells, and preparation and application thereof. The present disclosure also provides methods of treating cancer characterized by the presence of a solid tumor, by administering the pharmaceutical preparation. The terms "cancer characterized by the presence of a solid tumor" and "tumor" are used interchangeably herein.

The objects of the present disclosure are achieved by the following technical solutions:

A first aspect of the present disclosure provides an immune cell redirecting liposome. The immune cell redirecting liposome includes a liposome, a targeting antibody fragment and an immune cell binding antibody fragment loaded on the surface of the same liposome, the targeting antibody can specifically bind to the surface of target cells, and the immune effector cell binding antibody binds to immune effector cells.

Further, the immune cell redirecting liposome is capable of simultaneously or sequentially binding to immune effector cells and target cells, such that the immune effector cell would recognize target cells and activate immune effects
Further, in one single liposome nanoparticle, the molar ratio of the targeting antibody to the immune cell binding antibody is between (10/1 and 1/10).

Further, each liposome has about 1-1000 targeting antibody antibodies and 1-1000 immune cell binding antibodies anchored on the surface.

As exemplified in some embodiments of the present disclosure, the targeting antibody fragment and the immune cell binding antibody fragment are respectively conjugated to a lipid molecule and anchored on the liposome surface. In some embodiment, the targeting antibody and the immune cell binding antibody are conjugated to the maleimide group of the lipid. The targeting antibody and the immune cell binding antibody are respectively connected to the lipid molecule via a thioether bond.

Further, the target cells are selected from a group consisting of tumor cells, microorganisms, and microorganism-infected cells. Therefore, the antigen targeted by the targeting antibodies are selected from a group consisting of microbial antigens, tumor-related antigens, tumor cell surface specific antigens, a highly expressed antigen on the tumor cell surface, and a highly expressed antigen in a tumor tissue or a tumor blood vessel.

Further, the specific target molecule of the targeting antibody is selected from a group consisting of HER2, CD19, CD20, PSMA, Her2/neu, EGFR, LGR5 or PDL1.

In some embodiments of the present disclosure, the specific target molecule of the targeting antibody is a CD19 or a HER2.

Further, the immune cell is an effector cell capable of producing cytotoxicity, pro-apoptosis or clearance effects on target cells. The immune effector cell is selected from T lymphocyte, NK cell, NKT cell, monocyte, Dendritic cells, macrophage, or neutrophil.

Therefore, the immune cell binding antibody binds to antigens selected from those expressed by immune effector cells. Therefore, the immune cell redirecting liposomes can simultaneously or sequentially bind to tumor cells, lymphocytes or other immunocells. or a myeloid lineage cell. That is, the same liposome binds to a tumor cell and then a lymphocyte, or vice versa.

In some embodiments of the present disclosure, the immune effector cell is a T lymphocyte.

In some embodiments of the present disclosure, the antigen of the immune effector cell is CD3, and the immune cell binding antibody is a CD3 antibody.

Further, the immune cell antibody is selected from a CD3 antibody fragment, a PD1 antibody or fragment, , a CTLA4 antibody fragment, a CD40L antibody and fragment.

The T lymphocyte may be a naive T cell, or an in-vitro activated or expanded T cell, for example, an antigen-specific T lymphocyte, a tumor infiltrating lymphocyte, a cytotoxic T cell, a helper T cell, a lymphokine-activated killer cell (LAK cells), a γδ T cell, a chimeric antigen receptor T cell, or a T cell receptor chimeric T cell.

The immune cell activation or expansion may be achieved by antigen, antibody, polypeptide, polypeptide-major histocompatibility complex, small molecule, cytokine, immune checkpoint inhibitor or gene editing.

Further, the form of the targeting antibody and the immune cell binding antibody is selected from IgG, Fab' fragment, F(ab')2 fragment, Fab fragment, single-chain Fv fragment, minibody, nanobody, unibody, or diabody.

As exemplified in some embodiments of the present disclosure, the antibody is a Fab' fragment which is obtained after removing the Fc fragment and reducing the antibody. The antibody includes a light chain variable region and a heavy chain variable region.

As exemplified in some embodiments of the present disclosure, the targeting antibody is a Fab' fragment of a CD19 antibody, and the amino acid sequence of the light chain (LC) variable (VL) region is shown in SEQ ID NO. 1, specifically:

DIQMTQTTSSLSASLGDRVTISCRASQDIRNYLNWYQQKPDGTVKLLIYYTSRLHSG
VPSKFSGSGSGTDYSLTISNLEQEDIATYFCQQGNTLPWTFAGGTKLEIK.

The amino acid sequence of the heavy chain (HC) variable (VH) region is shown in SEQ ID NO. 2, specifically:

EVQLQQSGPELVKPGASMKISCKASGYSFTGYTMNWVKQSHGKNLEWMGLINPY

KGVSTYNQKFKDKATLTVDKSSSTAYMELLSLTSEDSAVYYCARSGYYGDSDWYF
DVWGQGTTLTVFS.

In addition, the targeting antibody may be an anti-Her2 antibody, and the LC sequence of the anti-Her2 antibody is shown in SEQ ID NO. 3, specifically:

DIQMTQSPSSLSASVGDRVTITCRASQDVNTAVAWYQQKPGKAPKLLIYSASFLYSGVP
SRFSGSRSGTDFTLTISSLQPEDFATYYCQQHYTTPPTFGQGTKVEIKRTVAAPSVFIFPPS
DEQLKSGTASVVCLLNNFYPREAKVQWKVDNALQSGNSQESVTEQDSKDSTYSLSSTL
TLSKADYEKHKVYACEVTHQGLSSPVTKSFNRGEC

The HC sequence of the anti-Her2 antibody is shown in SEQ ID NO. 4, specifically:

EVQLVESGGGLVQPGGSLRLSCAASGFNIKDTYIHWVRQAPGKGLEWVARIYPTNGYT
RYADSVKGRFTISADTSKNTAYLQMNSLRAEDTAVYYCSRWGGDGFAMDYWGQGTL
VTVSSASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTCAA

In addition, the targeting antibody may be an scFv fragment of an EGFR antibody, and the VH sequence of the scFv of the EGFR antibody is shown in SEQ ID NO. 5, specifically:

QVQLKQSGPGLVQPSQSLSITCTVSGFSLTNYGVHWVRQSPGKGLEWLGVIWSGG
NTDYNTPFTSRLSINKDNSKSQVFFKMNSLQSNDTAIYYCARALTYYDYEFAYWGQ
GTLVTVSA.

The VL sequence of the EGFR antibody is shown in SEQ ID NO. 6, specifically:

DILLTQSPVILSVSPGERVSFSCRASQSIGTNIHWYQQRTNGSPRLLIKYASESISGIPS
RFSGSGSGTDFTLSINSVESEDIADYYCQQNNNWPTTFGAGTKLELK.

The targeting antibody may be a PSMA antibody, and the VH sequence of the PSMA antibody is shown in SEQ ID NO. 7, specifically:

QVQLVESGGGLVKPGESLRLSCAASGFTFSDYYMYWVRQAPGKGLEWVAIISDGG
YYTYYSDIIKGRFTISRDNAKNSLYLQMNSLKAEDTAVYYCARGFPLLRHGAMDY
WGQGTLVTVSS.

The VL sequence of the PSMA antibody is shown in SEQ ID NO. 8, specifically:

DIQMTQSPSSLSASVGDRVTITCKASQNVDTNVAWYQQKPGQAPKSLIYSASYRYS
DVPSRFSGSASGTDFTLTISSVQSEDFATYYCQQYDSYPYTFGGGTKLEIK.

The target antibody may be a PDL1 antibody, and the HC sequence of the PDL1 antibody is shown in SEQ ID NO. 9, specifically:

EVQLLESGGG LVQPGGSLRL SCAASGFTFS SYIMMWVRQA PGKGLEWVSS 50
IYPSGGITFY ADTVKGRFTI SRDNSKNTLY LQMNSLRAED TAVYYCARIK 100
LGTVTTVDYW GQGTLVTVSS ASTKGPSVFP LAPSSKSTSG GTAALGCLVK 150
DYFPEPVTVS WNSGALTSGV HTFPAVLQSS GLYSLSSVVT VPSSSLGTQT 200
YICNVNHKPS NTKVDKKVEP KSCDKTHTCP PCPAPELLGG PSVFLFPPKP 250
KDTLMISRTP EVTCVVVDVS HEDPEVKFNW YVDGVEVHNA KTKPREEQYN 300
STYRVVSVLT VLHQDWLNGK EYKCKVSNKA LPAPIEKTIS KAKGQPREPQ 350
VYTLPPSRDE LTKNQVSLTC LVKGFYPSDI AVEWESNGQP ENNYKTTPPV 400
LDSDGSFFLY SKLTVDKSRW QQGNVFSCSV MHEALHNHYT QKSLSLSPGK 450.

The light chain sequence of the PDL1 antibody is shown in SEQ ID NO. 10, specifically:

QSALTQPASV SGSPGQSITI SCTGTSSDVG GYNYVSWYQQ HPGKAPKLMI 50
YDVSNRPSGV SNRFSGSKSG NTASLTISGL QAEDEADYYC SSYTSSSTRV 100
FGTGTKVTVL GQPKANPTVT LFPPSSEELQ ANKATLVCLI SDFYPGAVTV 150
AWKADGSPVK AGVETTKPSK QSNNKYAASS YLSLTPEQWK SHRSYSCQVT 200
HEGSTVEKTV APTECS.

As exemplified in some embodiments of the present disclosure, the immune cell binding antibody is a Fab' fragment of a CD3 antibody and the LC sequence of the anti-CD3 antibody Fab fragment is shown in SEQ ID NO. 11, specifically:

EVQLVESGGGLVQPGGSLRLSCAASGYSFTGYTMNWVRQAPGKGLEWVALINPYKGVT
TYADSVKGRFTISVDKSKNTAYLQMNSLRAEDTAVYYCARSGYYGDSDWYFDVWGQG
TLVTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKKVEPKSCDKTHTCAA

HC sequence of the CD3 antibody is shown in SEQ ID NO. 12, specifically:

DIQMTQSPSSLSASVGDRVTITCRASQDIRNYLNWYQQKPGKAPKLLIYYTSRLESGVPSR
FSGSGSGTDYTLTISSLQPEDFATYYCQQGNTLPWTFGQGTKVEIKRTVAAPSVFIFPPSDE
QLKSGTASVVCLLNNFYPREAKVQWKVDNALQSGNSQESVTEQDSKDSTYSLSSTLTLS
KADYEKHKVYACEVTHQGLSSPVTKSFNRGEC

The immune cell binding antibody may be a PD-1 antibody, and the VH sequence of the PD-1 antibody is shown in SEQ ID NO. 13, specifically:




.

The VL sequence of the PD-1 antibody is shown in SEQ ID NO. 14, specifically:

LEMAEIVLTQSPATLSLSPGERATLSCRASQSVSSYLAWYQQKPGQAPRLLIYDASN
RATGIPARFSGSGSGTDFTLTISSLEPEDFAVYYCQQSSNWPRTFGQGTKVEIK.

The present disclosure has no special limitation on the composition of the liposome, as long as it is a liposome. The components of the liposome may be selected from the group consisting of egg phospholipid, hydrogenated soybean phosphatidylcholine, hydrogenated egg phosphatidylcholine, dilauroylphosphatidylcholine, dimyristoylphosphatidylcholine, dipalmitoylphosphatidylcholine, distearoylphosphatidylcholine, 1-myristoyl-2-palmitoylphosphatidylcholine, 1-palmitoyl-2-stearoylphosphatidylcholine, 1-stearoyl-2-palmitoylphosphatidylcholine, 1-palmitoyl-2-oleylphosphatidylcholine, 1-stearoyl-2-linoleylphosphatidylcholine, dioleylphosphatidylcholine, hydrogenated dipalmitoylphospholipidcholine, distearoylphosphatidylcholine, dimyristoylphosphatidic acid, dimyristoylphosphatidic acid, dipalmitoylphosphatidic acid, dipalmitoylphosphatidic acid, distearoylphosphatidic acid, dimyristoyl phosphatidylethanolamine, dipalmitoylphosphatidylethanolamine, brain phosphatidylserine, dimyristoylphosphatidylserine, dipalmitoylphosphatidylserine, egg phosphatidylglycerol, dilauroylphosphatidyl glycerol, dimyristoylphosphatidyl glycerol, dipalmitoylphosphatidylglycerol, distearylphosphatidylglycerol, dioleoylphosphatidylglycerol, brain sphingomyelin, dipalmitoyl sphingomyelin or distearoyl sphingomyelin, cholesterol, dioleoxypropyltrimethylammonium chloride (DOTAP), dioleyl chloropropyl trimethylammonium chloride (DOTMA), dimethyldioctadecylammonium bromide (DDAB), dimethylaminoethane carbamoyl-cholesterol (DC-Chol), spermine-5-carboxyaminoacetic acid octacosylamide (DOGS), dioleosuccinylglycerol choline ester (DOSC), dioleyl chlorospermine carboxyl amide ethyl dimethyl propyl ammonium trifluoroacetate (DOSPA), Cholesterol and DSPE-PEG-2000 or a combination of two or more thereof.

As exemplified in some embodiments of the present disclosure, the components of the liposome include phosphatidylcholine, cholesterol, a lipid conjugated to a targeting antibody, and a lipid molecule linked to an immune cell binding antibody.

Further, the liposome may or may not contain an encapsulated drugs in the internal aqueous phase.

In some embodiments, the average number of the immune cell binding antibody per liposome is from 1 to 9. In some embodiments, the average number of the anti-CD3 fragment per liposome is from 1 to 8. In some embodiments, the average number of the anti-CD3 fragment per liposome is from 1 to 7. In some embodiments, the average number of the anti-CD3 fragment per liposome is from 1 to 6. In some embodiments, the average number of the anti-CD3 fragment per liposome is from 1 to 5. In some embodiments, the average number of the anti-CD3 fragment per liposome is from 1 to 4. In some embodiments, the average number of the anti-CD3 fragment per liposome is from 1 to 3. In some embodiments, the average number of the anti-CD3 fragment per liposome is from 1 to 2.

In some embodiments, the average number of the immune cell binding antibody fragment per liposome is from 2 to 9. In some embodiments, the average number of the anti-CD3 fragment per liposome is from 2 to 6. In some embodiments, the average number of the anti-CD3 fragment per liposome is from 3 to 9. In some embodiments, the average number of the anti-CD3 fragment per liposome is from 3 to 8. In some embodiments, the average number of the anti-CD3 fragment per liposome is from 3 to 6.

In some embodiments, the average valency of the immune cell binding antibody fragment per liposome is from 4 to 9. In some embodiments, the average number of the anti-CD3 fragment per liposome is from 4 to 8. In some embodiments, the average number of the anti-CD3 fragment per liposome is from 5 to 7. In some embodiments, the average number of the anti-CD3 fragment is 6.

In some embodiments, the average valency of the anti-HER2 antibody fragment per liposome is from 3 to 20. In some embodiments, the average valency of the anti-HER2 antibody fragment per liposome is from 3 to 18. In some embodiments, the average valency of the anti-HER2 antibody fragment valency per liposome is from 3 to 15. In some embodiments, the average valency of the anti-HER2 antibody fragment valency per liposome is from 3 to 12. In some embodiments, the average valency of the anti-HER2 antibody fragment valency per liposome is from 3 to 10.

In some embodiments, the average valency of the anti-HER2 antibody fragment per liposome is from 5 to 20. In some embodiments, the average valency of the anti-HER2 antibody fragment per liposome is from 6 to 20. In some embodiments, the average valency of the anti-HER2 antibody fragment per liposome is from 7 to 20. In some embodiments, the average valency of the anti-HER2 antibody fragment per liposome is from 8 to 20. In some embodiments, the average valency of the anti-HER2 antibody fragment per liposome is from 10 to 20.

In some embodiments, the average valency of the anti-HER2 antibody fragment per liposome is from 8 to 15. In some embodiments, the average valency of the anti-HER2 antibody fragment per liposome is from 9 to 12. In some embodiments, the average valency of the anti-HER2 antibody fragment per liposome is from 9 to 11. In some embodiments, the average valency of the anti-HER2 antibody is 10.

The liposomes may be loaded with one or more suitable compounds for immune modulation effects. The compounds may include one or more anti-cancer drugs, and/or immunomodulatory drugs. In some embodiments, the encapsulated immunomodulator drug includes resiquimod (also known as CD11301; R848; S28463; VML600), imiquimod (Aldara^{®}), MBS8, Guretolimod (DSP-0509), TransCon^{™}, GSK1795091, GSK2245035, VTX-2337, eritoran (E5564), SD-101, Selgantolimod, RO7119929, CpG1018, Vidutolimod (CMP-001), SHR2150, MGN1703, CYT003-QbG10, CPG-7909, Tilsotolimod, JNJ-64794964 (AL-034/TQ-A3334), BNT411, APR003, BDB001, or combinations of two or more thereof.

Additional immunomodulatory drugs suitable for encapsulation in liposomes are known in the art. *See, e.g.,* U.S. Patents 8,202,974, 9,050,376, 9,211,320, 9,248,170, 9,295,732, 9,458,184, 10,076,535, 10,259,793, and 11,026,964, and U.S. Patent Application Publications 2018/0360974, 2018/0085388, 2020/0316211, 2021/0059953, 2021/0371440, and 2022/0192997.

Additional drugs suitable for encapsulation in liposomes are known in the art. *See, e.g.,* U.S. Patents 8,597,654, 8,895,717, 9,326,953, 9,351,997, 9,789,193, 9,993,551, 10,376,519, 10,426,753, 10,618,905, 10,653,774, 10,654,831, and 11,096,900, and U.S. Patent Application Publications 2005/240131, 2020/0001110, 2020/0283406, 2021/0077397, 2022/0160729, and 2021/0275453.

In some embodiments, the encapsulated drugs include dasatinib. In some embodiments, the encapsulated drug includes resiquimod.

In some embodiments, the encapsulated drug is resiquimod at the resiquimod to lipid ratio of 1:10 to 1:100 (wt:wt), the resiquimod was encapsulated either in the inner aqueous of the liposome, or in the bilayer of the liposome.

In some embodiments, the ratio of resiquimod to lipid is 1:15 (wt:wt). In some embodiments, the ratio of resiquimod to lipid is 1:25. In some embodiments, the ratio of resiquimod to lipid is 1:50.

The overall concentration of R848 in the TRAFsome solution is between 0.5mg/mL to 1mg/mL While the particle size of the resiquimod loaded TRAFsome is about 80nm.

In some embodiments, the resiquimod was acted as an immune modulator in the TRAFsome in order to induce a proper immune response. This immune responses include the activation of the T cells and the pro-inflammatory cytokine secretion

In some embodiments, the TRAFsome loaded with resiquimod contained the following lipids.

**Table 1**

| **Lipid Compound** | **Molar ratio(%)** |
|---|---|
| HSPC | 55~60 |
| DSPE-PEG2000 | 1~5 |
| Cholesterol | 36~40 |
| Her2 binding antibody | 0~1 |
| CD3 binding antibody | 0~1 |

A third aspect of the present disclosure provides the use of the aforementioned T cell redirecting liposomes in preparing immunotherapeutic drugs and antitumor drugs.

Compared with the traditional technology, the present disclosure has the following beneficial effects:
The T cell redirecting liposome of the present disclosure has a mechanism similar to that of the multi-sepcific antibodies. The present disclosure further proposes to optimize the target specific immune cell activation effects by adjusting the density and relative proportions of the targeing antibody and immune cell binding antibody. Furthermore, the effects of liposome encapsulating drugs provide further immune modulation. Finally, the production, preparation and quality control of the multicellular liposome are also more feasible compared with multi-specific antibodies.

### Brief Description of The Drawings

FIG. 1: Schematic drawings of different T cell redirecting mechanisms, comparing bispecific T cell engager (BITE), dual affinity retargeting antibody (DART), and T-cell redirecting antibody fragment-anchored liposome (TRAFsome).
FIG. 2A: Reduced SDS-PAGE and non-reduced SDS-PAGE of full-length CD3 antibody, anti-CD3 F(ab')2, and anti-CD3 Fab' conjugated lipids. 2B: SDS-PAGE of full-length CD19 antibody, anti-CD19 antibody F(ab')2, and anti-CD19 Fab' conjugated lipids. 2C: SDS-PAGE images of anti-CD3 single-chain antibody (scFv), anti-CD3 single-chain antibody (scFv) conjugated lipids. 2D Estimated surface densities of antibodies on liposomes containing different amounts of Fab'.
FIG. 3A: Schematic illustration of anti-Her2 conjucated lipid (TL01). FIG. 3B: Schematic illustration of anti-CD3 conjucated lipid (TL02).
FIG.4: Evaluation of the T cell activation effects by TRAFsomes.
FIG.5: Evaluation of the T cell activation and effector function using a mixed lymphocyte reaction assay.
FIG.6: The anti-tumor effect of TRAFsomes in a Patient-derived Xenografts( Adrenocortical cancer, ACC) PBMC reconstituted mouse model.
FIG.7: Evaluation of the anti-tumor Effect of TRAFsomes in a N87 tumor model.
FIG.8: Evaluation of the anti-tumor Effect of the TRAFsomes in HER2 expression 4T1 Syngeneic tumor models.
FIG.9: Evaluation of the effect of dasatinib loaded TRAFsomes.
FIG.10: Examining the effect of immune cell binding Fab (anti-CD3) valency.
FIG.11: Evaluation of the effect of dasatinib loaded TRAFsome with Raji+-PBMC co-engraftment model.
FIG.12: TRAFsomes activities on γδT cells, IL-2 stimulated LAK cells and aCD3/aCD28 bead expanded T cells.

### Detailed Description of The Preferred Embodiments

Liposomes are artificially prepared bilayer vesicles formed by lipid molecules. The structure of a lipid molecule usually consists of a hydrophilic head, a hydrophobic tail, and a linker. In the aqueous medium, due to hydrophobic interactions, the hydrophobic tails of the lipid molecules are close to each other, and the hydrophilic heads are arranged facing toward the aqueous phase, forming a bilayer. Based on such a structure, hydrophilic drugs can be encapsulated in the internal aqueous phase (internal water phase) of the liposome, and hydrophobic drugs can be embedded in the lipid bilayer.

The targeting effect of the liposomes is achieved by incorporate ligands on the surface of liposomes. The ligand may be an antibody, a polypeptide, a small molecule, or the like. The ligand interacts with the target cell receptor. When antibody and antibody fragments are used as ligands, liposomes have better binding activity and specificity compared to small molecules and polypeptide ligands. Applying the principle of self-assembly of lipid molecules, it is also feasible to incorporate two or more types of ligands on the surface of one liposome.

Based on the antibody-modified liposome technology, the present disclosure enables a novel and unique killing mechanism and effect towards target cells through a clever combination of the targeting antibody and the immune cell binding antibody. The currently known multi-targeting liposome preparations use the combination of different ligands to improve the targeting delivery efficiency and selectivity towards target cells. The TRAFsome of the present disclosure also regulates physiological functions such as recognition, binding, and activation of the immune cells and promote their interaction with target cells. Therefore, the present disclosure provides a new mechanism for ligand-directed liposome targeting.

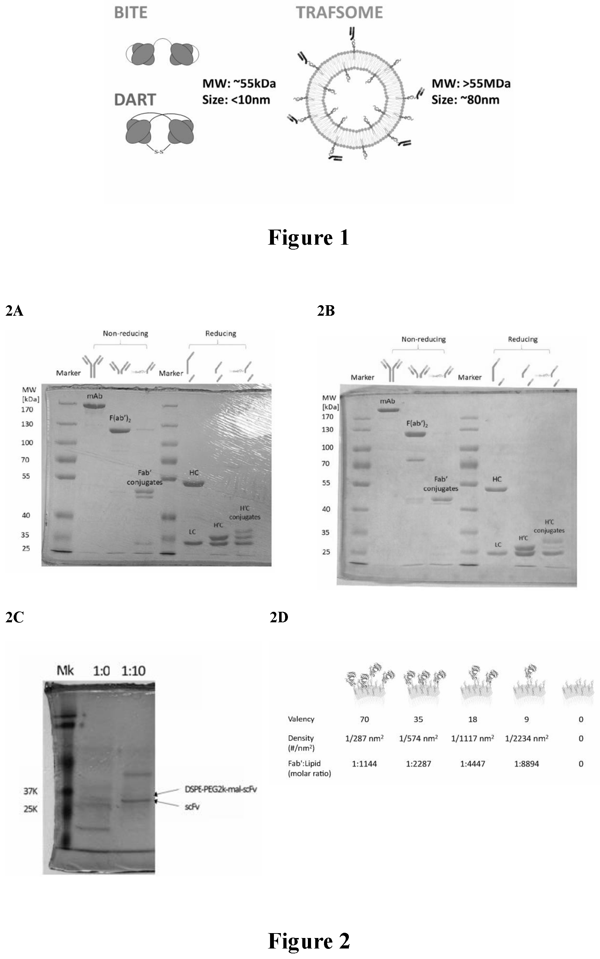

Existing strategies for multi-specific antibody complexes are illustrated in Figure. 1. They include molecules such as a bispecific T cell engager (BITE) or a dual affinity retargeting antibody (DART), both contained two binding domains at a 1:1 ratio. On the other hand, incorporating different binding domains on the surface of a liposome is not only more convenient, but also enables variations of the density (valency) and ratio of antibody bidning domains on each liposome (e.g., TRAFsome). Furthermore, T cell redirection liposomes are larger than BITEs and DARTs (typically about 5 nm in diameters) and may be 10 times as larger, the pharmacokinetic behavior and distribution patterns are much more favorable than those of currently known BiTe or DART. In addition, such construction of the TRAFsomes would avoid problems such as antibody mismatch and overcome defects in production.

Further, the TRAFsome obtained by the present disclosure is compatible to liposome drug-loading technology. By encapsulating the drug in the liposome, the targeting delivery and release of the drug is achieved, that is, the selectivity of the drug is improved, and the releasing site and timing of the drug can be changed. For example, the adverse effects of tumor immunotherapy include the production of cytokine storms in patients. Liposomes encapsulating immune modulators can achieve more focused immune modulation and minimize systemic immune over-reactions adding to the benefit of T cell re-directing described in the current disclosure.

The T cell redirecting liposomes containing the targeting antibody, the immune cell binding antibody and encapsulated immune modulator in the present disclosure can promote immune cells binding and recognition of target cells, and modulate the immune effector function of the immune cells. The liposome has a unique mechanism of in-vivo action, and has substantial advantage in production, preparation and activities compared with the clinically applied multi-specific antibodies..

The present methods entail the use of the T cell redirecting liposomes as immunotherapeutic and antitumor drugs, in the treatment of a tumor. As understood in the art, "treatment" is an approach for obtaining beneficial or desired results, including clinical results. Such results may include any one or more of alleviation or amelioration of one or more symptoms of cancer, diminishment of extent of the disease or disorder, stabilization of the state of the disease or disorder, delay or slowing of the disease or disorder, remission of the disease or disorder, and killing of at least one cancer cell targeted by the disclosed compositions or induce an immune response to cancer cells.

In some embodiments, the tumor is a Her2/neu positive (Her2/neu⁺) tumor. Her2/neu⁺ tumors produce larger than normal amounts of Her2/neu and include breast, ovarian, bladder, pancreatic, stomach, and esophageal cancers. In some embodiments, the Her2/neu⁺ tumor is breast cancer. Her2/neu is also commonly referred to as Erb-b2 receptor tyrosine kinase 2 (ERBB2), c-erbB-2, and human epidermal growth factor (EGF) receptor 2 (HER2). In some embodiments, the tumor can be HER2 low or HER2- expressing tumors.

In some embodiments, the encapsulated drugs include dasatinib. In some embodiments, the encapsulated drug includes resiquimod.

In some embodiments, the TRAFsome encapsulated resiquimod in a ratio of resiquimod to lipid from 1:10 to 1:100 (w:w), the resiquimod is encapsulated either in the inner aqueous of the liposome, or in the bilayer of the liposome.

In some embodiments, The concentration of R848 in the TRAFsome preparation is from 0.5mg/mL to 1mg/mL, the encapsulated efficiency(%) of R848 is above 90%.

In some embodiments, the TRAFsome loaded with resiquimod has the following lipids composition (table). wherase TL01 is a Her2 antibody conjugated lipid and TL02 is a CD3 antibody conjugated lipid .

**Table 2**

| **Lipid Compound** | **Molar ratio(%)** |
|---|---|
| HSPC | 55~60 |
| DSPE-PEG2000 | 1~5 |
| Cholesterol | 36~40 |
| TL01 | 0~1 |
| TL02 | 0~1 |

As exemplified in some embodiments, the technical solution of the present disclosure has the following advantages:
(1) the present disclosure starts from the preparation of a liposome, incorporating two antibody conjugated lipids on the same liposome, and encapsulating immune-modulators. The lipid composition and encapsulated drug contents may be adjusted according to clinical needs.
(2) two antibody fragment conjugated lipids TL01 and TL02 for tumor cell HER2 bindning and immune cell CD3 binding are incorporated. Fluorescently labeled TRAFsomes showed strong binding activity when incubating with Her2 positive cancer cells and CD3 positive PBMCs, and enabled immune cell mediated lysis of the cancer cells.
(3) The TRAFsomes can accommodate drug encapsulation. TRAFsomes encapsulating the immunoregulatory drug resiquimod have superior immune effector cell mediated tumor cell killing effect.

Before further describing the specific embodiments of the present disclosure, it should be understood that the scope of protection of the present disclosure is not limited to the following specific embodiments; it should also be understood that the terms used in the embodiments of the present disclosure are just for describing the specific embodiments instead of limiting the scope of the present disclosure. The test methods without specific conditions noted in the following embodiments are generally based on conventional conditions or the conditions recommended by the manufacturers.

When the numerical values are given by the embodiments, it is to be understood that the two endpoints of each numerical range and any one between the two may be selected unless otherwise stated. Unless otherwise defined, all technical and scientific terms used in the present disclosure have the same meaning as commonly understood by one skilled in the art. In addition to the specific method, equipment and material used in the embodiments, any method, equipment and material in the existing technology similar or equivalent to the method, equipment and material mentioned in the embodiments of the present disclosure may be used to realize the invention according to the grasp of the existing technology and the record of the invention by those skilled in the art.

Unless otherwise stated, the experimental methods, detection methods, and preparation methods disclosed in the present invention all employ conventional techniques of molecular biology, biochemistry, chromatin structure and analysis, analytical chemistry, cell culture, recombinant DNA technology in the technical field and related fields. These techniques are well described in the prior literature. For details, please see Sambrook et al. MOLECULAR CLONING: A LABORATORY MANUAL, Second edition, Cold Spring Harbor Laboratory Press, 1989 and Third edition, 2001; Ausubel et al., CURRENT PROTOCOLS IN MOLECULAR BIOLOGY, John Wiley & Sons, New York, 1987 and periodic updates; the series METHODS IN ENZYMOLOGY, Academic Press, San Diego; Wolfe, CHROMATIN STRUCTURE AND FUNCTION, Third Edition, Academic Press, San Diego, 1998; METHOD IN ENZYMOLOGY, Vol. 304, Chromatin (PMWassarman and APWolffe, eds.), Academic Press, San Diego, 1999; and METHODS IN MOLECULAR BIOLOGY, Vol. 119, Chromatin Protocols (PBBecker, ed.) Humana Press, Totowa, 1999, etc.

Unless otherwise specified, the experimental materials used in the following embodiments were purchased from conventional biochemical reagent stores.

### EMBODIMENT 1 Preparation of TRAFsomes containing anti-CD19 and anti-CD3 conjugated lipids

### 1. Preparation of F(ab')2 Fragments of Anti-CD19 Antibody and Anti-CD3 Antibody:

The F(ab')2 fragment is prepared by excising the Fc fragment of IgG by the digestion reaction of an immobilized enzyme. The human IgG and mouse IgG are digested by pepsin, and mouse IgG1 is digested by ficin. The specific steps are as follows: centrifuging 500 µl of antibody, and passing through a desalting column; adding the flow-through liquid to the immobilized enzyme, and mixing at 37° C.; incubating the pepsin for 4 h or incubating the ficin for 24 h, collecting the flow-through liquid by centrifuging at 5,000 g for 1 min, washing the immobilized enzyme with Protein A Binding Buffer and collecting the flow-through liquid, and merging the flow-through liquids to obtain the enzyme-digested product.

Mixing the enzyme-digested product at a constant temperature in Protein A column for 10 min, collecting the flow-through liquid by centrifuging, washing the protein A column with Protein A Binding Buffer and collecting the flow-through liquid. Merging the flow-through liquids to obtain the enzyme-digested product purified by Protein A column and with the large fragments of IgG and Fc removed. Dialyzing the above enzyme-digested product through a 50 KD dialysis bag, and the dialysis medium is PBS solution (pH=7.0). The dialysis is performed in the order of 2 h, 2 h and 16h to obtain a purified antibody F(ab')2 fragment with the Fc fragment removed.

It is confirmed by the traditional technology that the obtained anti-CD3 antibody F(ab')2 fragment is the full-length anti-CD3 antibody with the Fc fragment being removed. The amino acid sequence of the light chain variable region is shown in SEQ. ID. NO. 1, specifically:

DIQMTQTTSSLSASLGDRVTISCRASQDIRNYLNWYQQKPDGTVKLLIYYTSRLHSG
VPSKFSGSGSGTDYSLTISNLEQEDIATYFCQQGNTLPWTFAGGTKLEIK.

The amino acid sequence of the heavy chain variable region is shown in SEQ ID NO. 2, specifically:

EVQLQQSGPELVKPGASMKISCKASGYSFTGYTMNWVKQSHGKNLEWMGLINPY
KGVSTYNQKFKDKATLTVDKSSSTAYMELLSLTSEDSAVYYCARSGYYGDSDWYF
DVWGQGTTLTVFS.

### 2. Preparation of the anti-CD19 conjugated lipid and anti-CD3 conjugated lipid.

The antibody F(ab')2 fragment is reduced to Fab' fragment by using β-mercaptoethylamine as a reducing agent. The Fab' fragment is chemically linked to the maleimide group of DSPE-PEG2000-Mal. The specific steps are as follows: mixing the F(ab')2 fragment (1-10 mg/ml) with 50 mM EDTA-containing β-mercaptoethylamine, reacting at 37° C. under the protection of N2 with shaking for 90 min; centrifuging, passing through a desalting column and collecting the flow-through liquid. Fab', the reduction product of the antibody F(ab')2 fragment from which β-mercaptoethylamine is removed, is obtained. Then, adding 30 mM HEPES solution containing 600 µM DSPE-PEG2000-Mal micelle, so that the molar ratio of Fab' to DSPE-PEG2000-Mal is 1:1, and the reaction is performed at 10° C. under the protection of N2 with shaking for 16 h. The process monitored SDS-PAGE are shown in Figure. 2A and 2B.

### 3. Preparation of Anti-CD3 scFv conjugated lipids

The antibody scFv fragment containing free sulfhydryl group was obtained by using tricarboxyethyl phosphine (TCEP) as a reducing agent, and chemically linked to the maleimide group of DSPE-PEG2000-Mal. The specific steps are as follows: mixing 800 µl of scFv fragment (1-10 mg/ml) with HEPES solution containing 1.2 mM TCEP, so that the molar ratio of scFv to TCEP is 1:5, then adding 30mM HEPES solution containing 600 µM DSPE-PEG2000-Mal micelle immediately, and reacting at 10° C. under the protection of N2 with shaking for 16 h; The process as monitored by SDS-PAGE is shown in FIG. 2C.

### 4. Preparation of Anti-CD19 and Anti-CD3 TRAFsomes

Liposomes made of HSPC, Cholesterol and DSPE-PEG-2000 were incubated with the antibody conjugated lipids for the preparation of TRAFsomes. The resulted TRAFsomes were washed 5 times by 300 KD ultrafiltration. The particle size of the liposomes is about 80-90 nm, and the PDI is about 0.1, as measured by dynamic light scattering. The average amount of Fab' copies per liposome was calculated by dividing the incorporated Fab' mole concentration with the liposome mole concentration (assuming 64 million Dalton MW for a large unilamellar vesicle (LUV)). Based on these copy numbers, the corresponding surface density of the Fab' on the liposome surface was estimated (FIG. 2D).

### EMBODIMENT 2 Preparation of TRAFsomes containing anti-HER2 and anti-CD3 conjugated lipids (TL01 and TL02)

A CHO cell line was established to express anti HER2 Fab. The sequence of the anti-HER2 Fab fragment has the following sequence:
**Heave-Chain:** 
EVQLVESGGGLVQPGGSLRLSCAASGFNIKDTYIHWVRQAPGKGLEWVARIY
PTNGYTRYADSVKGRFTISADTSKNTAYLQMNSLRAEDTAVYYCSRWGGDGFAMDYW
GQGTLVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSNTKVD
KKVEPKSCDKHTCAA
**Light-Chain:**

DIQMTQSPSSLSASVGDRVTITCRASQDVNTAVAWYQQKPGKAPKLLIYSASF
LYSGVPSRFSGSRSGTDFTLTISSLQPEDFATYYCQQHYTTPPTFGQGTKVEIK
RTVAAPSVFIFPPSDEQLKSGTASVVCLLNNFYPREAKVQWKVDNALQSGNS
QESVTEQDSKDSTYSLSSTLTLSKADYEKHKVYACEVTHQGLSSPVTKSFNR
GEC

A stable expression CHO cell line was also established to express anti-CD3 Fabs. The sequence is disclosed as following:
**Heavy chain:** 
DIQMTQSPSSLSASVGDRVTITCRASQDIRNYLNWYQQKPGKAPKLLIYYTSRLESGVPS
RFSGSGSGTDYTLTISSLQPEDFATYYCQQGNTLPWTFGQGTKVEIKRTVAAPSVFIFPPS
DEQLKSGTASVVCLLNNFYPREAKVQWKVDNALQSGNSQESVTEQDSKDSTYSLSSTL
TLSKADYEKHKVYACEVTHQGLSSPVTKSFNRGEC
**Light chain:**

EVQLVESGGGLVQPGGSLRLSCAASGYSFTGYTMNWVRQAPGKGLEWVALINPYKGV
TTYADSVKGRFTISVDKSKNTAYLQMNSLRAEDTAVYYCARSGYYGDSDWYFDVWGQ
GTLVTVSSASTKGPSVFPLAPSSKSTSGGTAALGCLVKDYFPEPVTVSWNSGALTSGVHT
FPAVLQSSGLYSLSSVVTVPSSSLGTQTYICNVNHKPSNTKVDKKVEPKSCDKTHTCAA

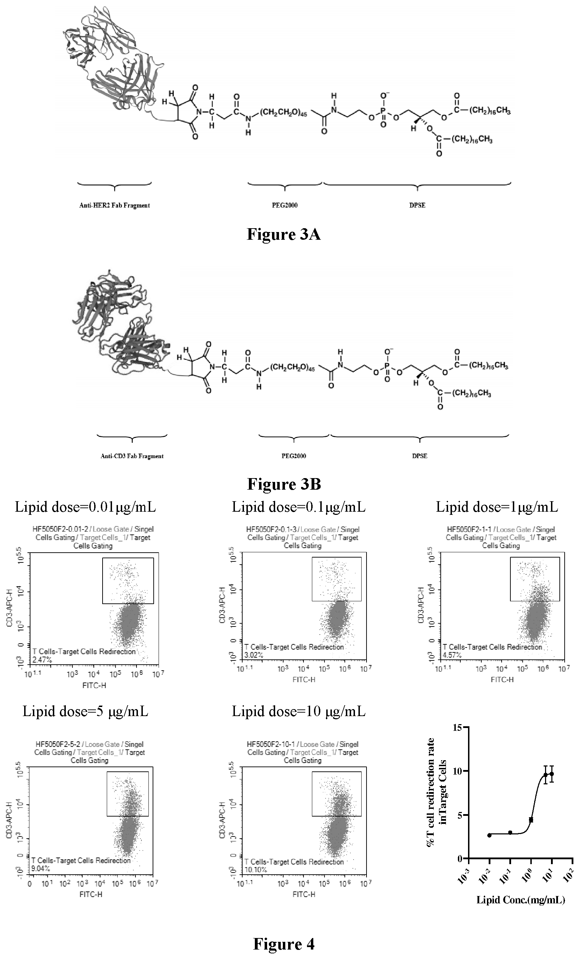

The anti-HER2 and anti-CD3 Fabs expressed and purified were then chemically linked to the maleimide group of DSG-PEG2000-Mal. The products were named as TL01 (anti-HER2 conjugated lipid) and TL02 (anti-CD3 conjugated lipid), respectively. The structure of TL01 and the TL02 were shown as Fig 3A and Fig 3B.

TRAFsomes containing TL01 and TL02 were made with the following compositions:

**Table 3**

| | Molar ratio | Weight |
|---|---|---|
| HSPC | 59.011 | 42.9 |
| DSPE-PEG2000 | 1.101 | 2.86 |
| Cholesterol | 39.874 | 14.3 |
| TL01 | 0.086 | 3.4 |
| TL02 | 0.005 | 4.0 |

The procedure to make TL01 and TL02 incorporated TRAFsome contain the following steps :
1) Weigh 42.9 g of hydrogenated soybean phosphatidylcholine (HSPC, molecular weight 783.8), 2.86 g of distearoylphosphatidylethanolamine-polyethylene glycol 2000 (DSPE-PEG2000), and 14.3 g of cholesterol. Dissolve them in 150 milliliters of ethanol and mix them in a 65°C water bath to obtain an ethanol lipid mixture.
2) Add the ethanol mixture obtained in step (1) into 2000 milliliters of ammonium sulfate buffer solution (pH 5.0, composed of 250 mM ammonium sulfate and water) and incubate in a 65°C water bath for 30 minutes to obtain liposomal vesicles.
3) Extrude the liposomal vesicles obtained in step (2) through polycarbonate membranes to finally obtain liposomes with an average particle size of about 75 nm in an aqueous phase.
4) Dialyze the liposomes obtained in step (3) through a 10,000-dalton cutoff dialysis membrane in a 10mM HEPES and 0.9% (w/w) NaCl solution with a pH of 6-8. The resulting liposomes have a pH and ion gradient between the inner and outer water phases of the bilayer.
5) 120.0 g TL01 solution (C=3.4mg/mL) and 59.5g TL02 solution (C=4.0mg/mL) were added into the blank liposome solution made at step (4). After raising the temperature to 60°C for 30 mins, The suspension was dialysis to purify the TRAFsomes and the size distribution was found to be about 80-90 nm, and the PDI is about 0.1.

### EMBODIMENT 3 Preparation of Resiquimod loaded TRAFsomes

The TRAFsomes containing the target binding antibodies and immune cell binding antibodies may also encapsulate immune modulators to add the desired efficacy while reduce toxicities. The drug encapsulation procedure into premade TRAFsomes is as follows:2000g Resiquimod was dissolved in 100mM pH5.5 sodium acetate buffer solution. The concentration of Resiquimod solution is 2mg/mL. Add the Resiquimod solution to preformed TRAFsomes and incubate for 30 mi. The encapsulation efficiency (EE) of Resiquimod was between 85% and 100%.

### EMBODIMENT 4 Evaluation of the T cell activation effects by TRAFsomes

The T cell redirection and activation effect of the present disclosure was examined using HER2-positive N87 cells as the target cells and CD3-positive Jurkat cells as the model T cell. A total number of 1×105 N87 cells and a total number of 1×105 of Jurkat cells were used in each reaction/plate. The cell mixtures were placed at 37 °C incubator for overnight (or 16hs). After then collected the cells and placing in a flow tube, centrifuging at 500×g for 5min. Jurkat cells were stained with antibody (APC anti-human CD3 Antibody, Cat#300311, PE/Cyanine7 anti-human CD25, Cat#356108, PE anti-human CD69 Antibody, Cat# 310906). After wishing the cells for 2 times, flow cytometry were employed to evaluate the Jurkart-N87 cell-cell conjugation (CD3-APC and CFSE-FITC fluorescence double positive). The results are shown in Figure 4.

### EMBODIMENT 5 Evaluation of the T cell activation and effector function using a mixed lymphocyte reaction assay.

To evaluate the TRAFsome mediated effector cells function, we employed a mixed lymphocyte reaction assay. Target cells (HER2-expression cells: N87, SK-Br-3 and BT474 cells line) and Effector cells (fresh isolated PBMCs from different donors) are used to investigate the cytotoxic activities of immune effector cells induced by Resiquimod loaded TRAFsome.

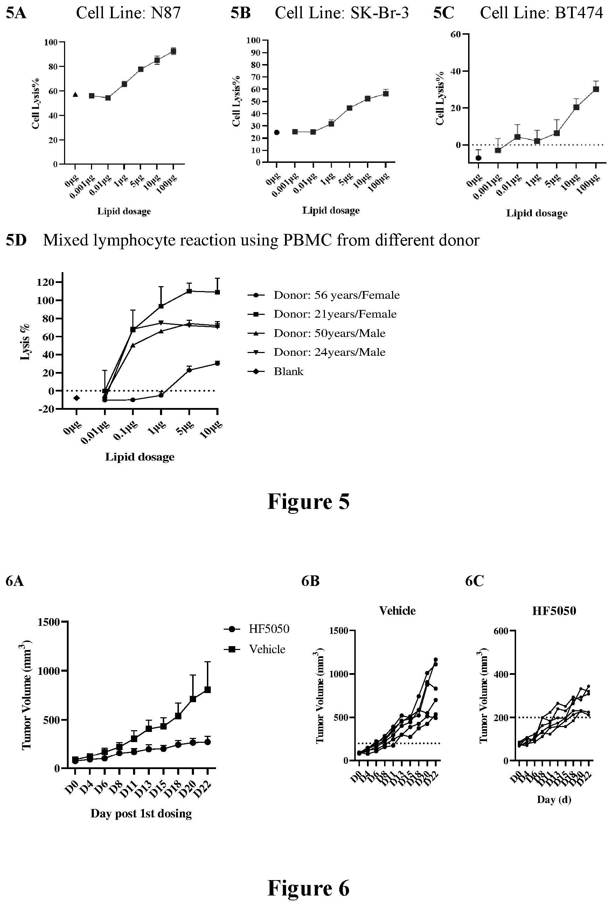

Specifically, effector cells and target cells are resuspended in phenol red-free complete medium, and the cell concentrations are 2×106 cells/ml and 5×105 cells/ml, respectively. Adding 100 µl effector cells and 100 µl target cells to a 96-well plate and mixing by pipetting to plate the cells. At this time, the ratio of target cells to effector cells is 1:5. Adding 10 µl of TRAFsome of different concentrations to each well, and mixing by pipetting. Placing the 96-well plate in a cell incubator, incubating for 24 h. 45 min before the end of the incubation, adding 10 µl lysate to the volume-correcting control well and the targeting cell maximum lysis well. After the incubation, centrifuging the 96-well plate at 250 g for 4 min. After centrifugation, pipetting 50 µl of the supernatant to a new 96-well plate, adding 50 µl of LDH substrate to the new well plate, and incubating at 22° C. for 15 min. After incubation, detecting the absorbance in a microplate reader at a wavelength of 490 nm. The results are shown in Figure 5

These experimental data fully demonstrate that the TRAFsomes of the present disclosure can effectively mediate the effector cells to effectively kill the target cells. and the effector cells may include unstimulated lymphocytes, in-vitro amplified lymphocytes, in-vitro activated lymphocytes, in-vitro induced differentiated lymphocytes. The cytotoxic effect is dependent on cell lysis and is dose-dependent.

### EMBODIMENT 6 The anti-tumor effect of TRAFsomes in a Patient-derived Xenografts( Adrenocortical cancer, ACC) PBMC reconstituted mouse model

The anti-tumor activities of immune-modulator loaded TRAFsome were tested in a Adrenocortical cancer patient derived Xenografts+PBMC co-engraftment model using NCG mice.

After injected with hu-PBMC for immune reconstituted for about 21 days, 12 mice were subcutaneously injected with Adrenocortical cancer patient derived cells and randomly divided into 2 groups, named as HF5050 group and Vehicle group. For the HF5050 group, mice received TRAFsome every three days starting at 7 days after tumor engraftment while Vehicle group were treated with PBS. Immune-modulator loaded TRAFsome were given at a Resiquimod dose of 0.28 mpK, the treatment were last for 3 weeks, the individual tumor growth curve were collected and the anti-tumor growth effect were evaluated. The individual tumor growth curves were plotted in FIG.6B, FIG.6C and summarized in FIG. 6A (mean ± SEM, n=6). Statistics were performed by comparing HF5050 group to the Vehicle control with t-test.

Encouragingly, the HF5050 showed effectively anti-tumor growth effect with TGI=44% after 8th dosing.

### Embodiment 7 Evaluation of the anti-tumor Effect of TRAFsomes in a N87 tumor model.

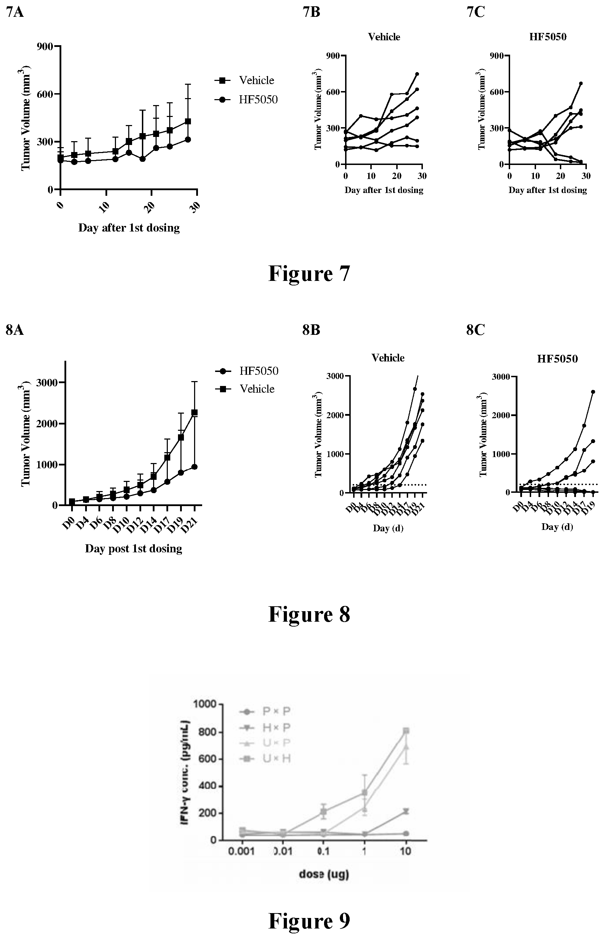

The anti-tumor activities of MTLs were tested in a N87-PBMC co-engraftment model using NOD/ShiLtJGpt-Prkdcem26Cd52Il2rgem26Cd22B2mem21Cd4/Gpt gene knock-out mice. 18 mice were subcutaneously injected with N87 cells and injected with hu-PBMC for immune reconstituted for about 14 days. Then 12 mice were randomly selected and divided into 2 groups, named as HF5050 group and Vehicle group. For the HF5050 group, mice received immune-modulator loaded TRAFsome every three days starting at 14 days after tumor engraftment while Vehicle group were treated with PBS. Immune-modulator loaded TRAFsome were given at a Resiquimod dose of 0.08 mpK, the treatment were last for 3 weeks, the individual tumor growth curve were collected and the anti-tumor growth effect were evaluated. The individual tumor growth curves were plotted in FIG.7B, FIG.7C and summarized in FIG. 7A (mean ± SEM, n=6). Statistics were performed by comparing HF5050 group to the Vehicle control with t-test.

### Embodiment 8. Evaluation of the anti-tumor Effect of the TRAFsomes in HER2 expression 4T1 Syngeneic tumor models.

The anti-tumor activities of TRAFsomes were tested in a syngeneic tumor models. The CD3ε was humanized in Balb/C mice and the 4T1 cells were HER2 expressing cell. 12 mice were subcutaneously injected with HER2 expressing cell and randomly divided into 2 groups, named as HF5050 group and Vehicle group. For the HF5050 group, mice received resiquimod loaded TRAFsome every three days starting at 7 days after tumor engraftment while Vehicle group were treated with PBS. The Resiquimod dose was given at 0.28 mpK, the treatment lasted for 3 weeks, the individual tumor growth curve were collected and the anti-tumor growth effect were evaluated. The individual tumor growth curves were plotted in FIG.8A, FIG.8B and summarized in FIG.8C (mean ± SEM, n=6). Statistics were performed by comparing HF5050 group to the Vehicle control with t-test.

### EMBODIMENT 9 Evaluation of the effect of dasatinib loaded TRAFsomes

The drug dasatinib encapsualated TRAFsomes are prepared and the effector cell activation effects were evaluated.

As an example, effector cells (unstimulated lymphocytes) and target cells (Raji cells) are resuspended in phenol red-free complete medium, and the cell concentrations are 1*10^6 cells/ml and 1.11*10^5 cells/ml, respectively. Adding 100 µl effector cells and 90 µl target cells to a 96-well plate and mixing by pipetting to plate the cells. At this time, the ratio of target cells to effector cells is 1:10. Adding 10 µl of drug encapsulated liposomes with different dasatinib concentrations and same lipid concentrations to each well, and mixing by pipetting. Placing the 96-well plate in a cell incubator, incubating for 24 h. 45 min before the end of the incubation, adding 10 µl lysate to the volume-correcting control well and the targeting cell maximum lysis well. After the incubation, centrifuging the 96-well plate at 250 g for 4 min. After centrifugation, pipetting 50 µl of the supernatant to a new 96-well plate, adding 50 µl of LDH substrate to the new well plate, and incubating at 22° C. for 15 min. After incubation, detecting the absorbance in a microplate reader at a wavelength of 490 nm.

The result shown in FIG. 9 demonstrates that dasatinib encapsulated TRAFsomes are effectve in directing various effector cell functions towards target cells.

### EMBODIMENT 10 Examining the effect of immune cell binding Fab (anti-CD3) valency

Liposomes (i.e., LUVs) with 80-90 nm mean diameters were prepared and anti-CD3 conjugated lipid (TL02) were incorporated as described. The resulted anti-CD3 copy number per liposome was estimated based on the total antibody concentration vs lipid concentration as described previously (Li et al., Pharm. Res. 38(9):1593-1600 (2021)). Briefly, since the liposomes were all about 80 nm in diameter and contain a single bilayer, the lipid mass per liposome was estimated to be 64 million Dalton. The molar ratio of antibody to liposome was reported as the antibody average valency can be estimated in accordance with standard techniques such as described for example, in Xie et al., Mabs 14(1):2115205 (2022), the contents of which are incorporated by reference in their entirety.

**Table 4**

| Valency | 70 | 35 | 18 | 9 | 6 | 5 | 4 | 3 | 2 | 1 |
|---|---|---|---|---|---|---|---|---|---|---|
| Density (#/nm²) | 1/287 | 1/574 | 1/ 1117 | 1/ 2234 | 1/3351 | 1/4021 | 1/5026.5 | 1/6702 | 1/ 10053 | 1/ 20106 |
| anti-CD3 Fab':lipid (molar ratio) | 1: 1144 | 1: 2287 | 1: 4447 | 1: 8894 | 1: 17788 | 1: 16009 | 1: 20011 | 1: 26682 | 1: 40023 | 1 : 80046 |

Since antibody conjugated lipids are free to diffuse laterally, their interactions with target cells could be multivalent. In fact, the radius of a TCR density domain was reported to be only about 1-3 nm on naive T cells and 35-70 nm on activated T cells (Lillemeier et al., Nat. Immunol. 11(1):90-96 (2010)). Therefore, anti-CD3 are more efficient when present on beads, even though most bead conjugated anti-CD3 are fixed with very limited degree of freedom to cluster. Interestingly, a recent study reported that anti-CD3 conjugated quantum dots with sizes smaller than 50 nm could not activate naive T cells, but they had better activity toward antigen-experienced T cells, probably because of preexisting TCR clustering (Lo et al., J. Immunol. 191(10):5107-5114 (2013)).

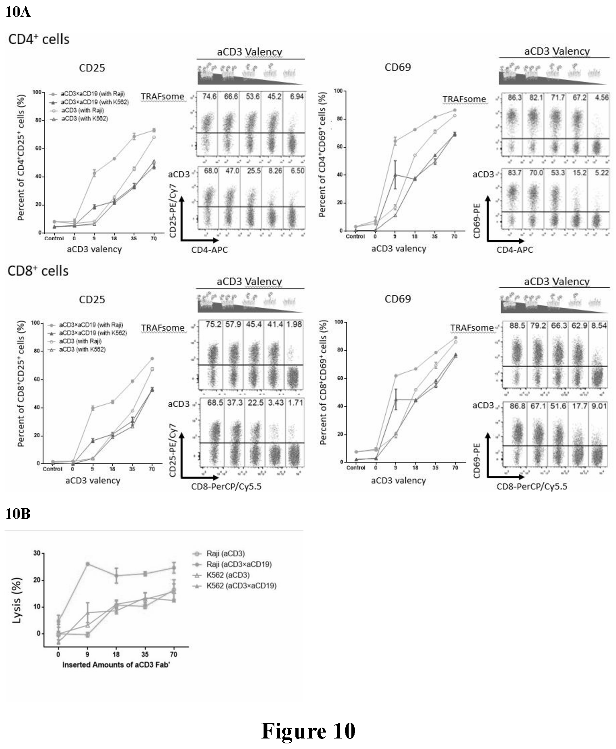

As shown in FIG. 10A, TRAFsomes with increasing anti-CD3 Fab' per liposome resulted in increasing amounts of CD25- and CD69-expressing T cells. aCD3 TRAFsomes with a valency of 9 or 18 caused about 20% of CD8+ T cells to become CD25+ and about 40% of CD8+ T cells to become CD69+. Increasing the CD3 valency to 35 or 70 further increased the percentage of CD25+ and CD69+ CD8+ T cells. TRAFsomes containing both anti-CD3 and targeting antibodies resulted in elevated levels of T cell activation in the presence of target cells. TRAFsomes with an anti-CD3 valency of 9 or 18 caused about 40% of CD8+ T cells to become CD25+ and about 60% of CD8+ T cells to become CD69+. Increasing the CD3 valency to 35 or 70 further increased the percentage of CD25+ and CD69+ CD8+ T cells; These data suggest that fewer anti-CD3 may be required when the TRAFsomes contained both targeting antibodies and immune cell binding antibodies.

Sinceliposomes containing only anti-CD3 Fab' caused some degree of T cell activation when there were more than 18 copies per liposome. Therefore, without being bound by theory, limiting the number of anti-CD3 Fab' per TRAFsomeL would minimize non-specific T cell activation. As illustrated in FIG. 10B, TRAFsomea containing only 9 anti-CD3 triggered significant Raji cell lysis, but very little K562 cell lysis. On the other hand, anti-CD3 liposomes with higher copies of anti-CD3 Fab' triggered cytotoxicity toward both cells independent of CD19 expression.

### EMBODIMENT 11 Evaluation of the effect of dasatinib loaded TRAFsome with Raji⁺-PBMC co-engraftment model

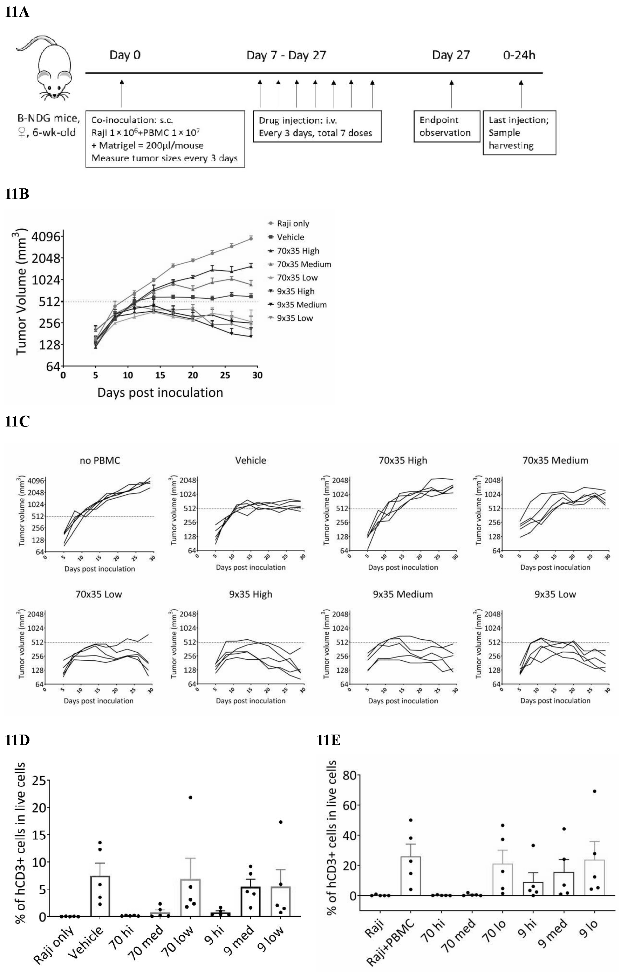

The anti-tumor activities of TRAFsomes were tested in a Raji⁺-PBMC co-engraftment model using NOD*-Prkdc^{scid}IL2rg*^{tm1} mice (FIG. 11A). NOD-Prkdc^{scid} IL2rg^{tm1} immunodeficient mice were subcutaneously injected with Raji cells (1×10⁶) and PBMCs (1×10⁷) mixed in Matrigel. Raji cell inoculated mice without human PBMC were included as controls. 9 × 35 TRAFsomes and 70 × 35 TRAFsomes were injected every three days starting at 7 days after tumor engraftment. Both TRAFsomes were given in three doses. Based on the in vitro data, the dose responses of TRAFsomes with either 9 or 70 anti-CD3 valences were compared (Table 5). The CD19⁺ Raji cells were co-engrafted with PBMC in Matrigel. These Raji⁺-PBMC co-engrafted tumors usually grew slower than tumors with Raji cells and no PBMCs because of the immune system reconstitution. The engrafted tumors were allowed to reach ~200 mm³ before treatments started. The individual tumor growth curves were plotted in FIG. 11C and summarized in FIG. 11B (mean ± SEM, n=5). Statistics were performed by comparing each group to the vehicle control. Encouragingly, the 9 × 35 TRAFsomes at the low dose of 65 µg lipid per injection effectively shrank the established tumor. Higher doses of the 9 × 35 TRAFsomes also resulted in reduction of tumor volume. Treatment of 70 × 35 TRAFsoms at the low dose of 63 µg lipid per injection resulted in similar tumor reductions as the 9 × 35 TRAFsomes doses. Strikingly, the treatments of 70 × 35 TRAFsome at the doses of 133 µg and 535 µg lipid (medium and high dose) had little to no anti-tumor effect. In fact, the tumors grew quickly, almost as fast as those in the mice that received tumors with Raji cells and no PBMCs.

**Table 5**

| Group (n=5) | Dose: aCD3-Fab'/Lipid (µm per mouse) | | |
|---|---|---|---|
| | Low | Medium | High |
| 70 x 35 | 7/33 | 28/133 | 112/535 |
| 9x35 | 1.76/65 | 7/260 | 28/1,1040 |
| Control | (1) Raji only | | |
| | (2) Raji + PBMC + PBS | | |

The mice were sacrificed after the last dose and the remaining human CD3+ T cells in their spleens were analyzed (FIG. 11D). There were very few human T cells remaining in 70 × 35 TRAFsome high- and medium-dose groups. The human T cell numbers in 70 × 35 TRAFsome low dose and 9 × 35 MTL medium- and low-dose groups were nearly as high as those in the vehicle control group. The 9 × 35 TRAFsome high-dose group was a little different, with fewer splenic human T cell remaining, but the tumor growth was much slower.

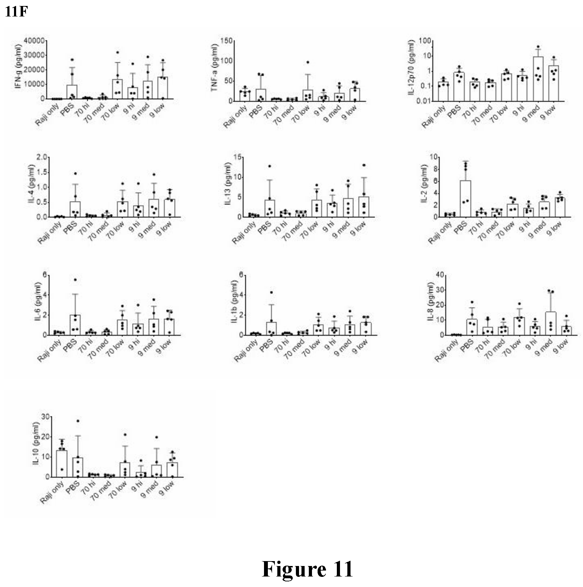

Blood samples collected on day 26 (24 hr before the last injection) and day 27 (3 hr after the last injection) were also analyzed. The percentages of circulating human CD45+ CD3+ in total leukocytes are shown in FIG. 11E, and the human inflammatory cytokine levels in plasma are shown in FIGs. 11F (*p < .05; **p < .01; ***p < .001; ****p < .0001, by two-way ANOVA combined with Tukey's multiple comparison test). Notably, the 9 × 35 TRAFsomes high-dose group had decent levels of human T cell numbers and various inflammatory cytokine levels in the plasma.

Without being bound by theory, treatment of mice with TRAFsome with an anti-CD3 valency of 70, may have caused T cell depletion or tolerance. Multi-valent CD3 ligation without IL-2 co-stimulation has been shown to cause T cell anergy, which could lead to failures in T cell-based immunotherapy (Duré and Macian, Mol. Immunol. 46(5):999-1006 (2009); Lechler et al., Immunology 103(3):262-269 (2001)). Complexed T cell engagers that contain more than one copies of anti-CD3 may be especially of concern (Zhukovsky et al., Curr. Opin. Immunol. 40:24-35 (2016); Vafa and Trinklein, Front. Oncol. 10(446):1-7 (2020)). The data disclose herein demonstrates that it is possible to alleviate the problem by reducing the anti-CD3 valency, and/or by incorporating immunomodulator inside the liposomes. The engagement of a wider repertoire of T cells including γδ T cells may also be beneficial, since there may be preexisting tumor evasion mechanisms that compromise tumor antigen-specific T cells (Garcia-Lora et al., J. Cell. Physiol. 195(3):346-355 (2003)).

### Embodiment 12 Drug Encapsulation in TRAFsomes

The TRAFsomes may be loaded with one or more suitable drugs. Encapsulated drugs may include one or more anti-cancer drugs, and/or immunomodulatory drugs. In some embodiments, the encapsulated immunomodulator drug includes resiquimod (also known as CD11301; R848; S28463; VML600), imiquimod (Aldara^{®}), MBS8, Guretolimod (DSP-0509), TransConTM, GSK1795091, GSK2245035, VTX-2337, eritoran (E5564), SD-101, glucopyranosyl lipid adjuvant-stable emulsion (GLA-SE), Selgantolimod, RO7119929, CpG1018, Vidutolimod (CMP-001), SHR2150, MGN1703, CYT003-QbG10, CPG-7909, Tilsotolimod, JNJ-64794964 (AL-034/TQ-A3334), BNT411, APR003, BDB001, or combinations of two or more thereof.

Additional immunomodulatory drugs suitable for encapsulation in liposomes are known in the art. See, e.g., U.S. Patents 8,202,974, 9,050,376, 9,211,320, 9,248,170, 9,295,732, 9,458,184, 10,076,535, 10,259,793, and 11,026,964, and U.S. Patent Application Publications 2018/0360974, 2018/0085388, 2020/0316211, 2021/0059953, 2021/0371440, and 2022/0192997.

In some embodiments, the encapsulated anti-cancer drug includes cyclophosphamide (Cytoxan^{®}; Cytoxan Lyophilized ^{®}), Axitinib (Inlyta^{®}), Bevacizumab (Avastin^{®}) Cabozantinib (Cometrig^{®}), 5,6-dimethylxanthenone-4-acetic acid (DMXAA), combretastatin A-4 phosphate (CA4P), curcumin, doxorubicin (Dox) (Doxil^{®}, Caelyx^{®}, Lipo-Dox^{®}, Myocet^{®}, Zolsketil^{®}), mitomycin-C lipidic prodrug (MLP) (Promitil^{®}), alendronate (Ald), cytarabine (DepoCyt^{®}), cytarabine and daunorubicin (Vyxeos^{®}), daunorubicin (DNR, DaunoXome^{®}), all-trans retinoic acid (ATRA), mitoxantrone (MXT, PLM60), paclitaxel (PCX, EndoTAG^{®}), Irinotecan (Onivyde^{®}/Nal-IRI), Vincristine (Marqibo^{®}), Mifamurtide (Mepact^{®}), cis-bis-neodecanoato-trans-R,R-1,2-diaminocyclohexane platinum II (L-NDDP, Aroplatin^{™}), Cisplatin (LiPlaCis, LipoplatinTM, SPI-077), or combinations of two or more thereof.

Additional chemotherapy drugs suitable for encapsulation in liposomes are known in the art. See, e.g., U.S. Patents 8,597,654, 8,895,717, 9,326,953, 9,351,997, 9,789,193, 9,993,551, 10,376,519, 10,426,753, 10,618,905, 10,653,774, 10,654,831, and 11,096,900, and U.S. Patent Application Publications 2005/240131, 2020/0001110, 2020/0283406, 2021/0077397, 2022/0160729, and 2021/0275453.

In some embodiments, the encapsulated drug includes dasatinib and resiquimod.

The encapsulation of dasatinib inside TRAFsomes was examined to improve CD8+ CTL activities. Dasatinib is a broad kinase inhibitor targeting various Src family kinases but was also found to be involved in T cell effector functions. The effects of unloaded MTLs mixed with free dasatinib versus MTLs loaded with dasatinib was compared on activated CD4+ and CD8+ T cells, while T cells stimulated with both anti-CD3 Fab' and anti-CD28 Fab' were used as the positive control. Dasatinib at the dose of 1-10 nM could significantly improve CD8+ T cell activation with limited effects on CD4+ T cells. Plate-bound anti-CD3 mAb (UCHT1) at equivalent molar concentration (5.6 nmol/L) plus soluble anti-CD28 mAb (2ug/ml) were used as the positive control. Data shown are mean ± SEM (n = 3). T cell activation profiles of MTLs and dasatinib-loaded MTLs was compared in the presence of CD19+ Raji cells and wildtype K562 cells (CD19 negative). While the nonspecific T cell activation triggered by MTLs at the lipid dose of 5 µg/ml with K562 were almost as high as those with Raji cell, the addition of dasatinib-loaded MTLs notably reduced the nonspecific activation. Lipid concentration was fixed at 5ug/ml. Dasatinib concentration fixed at 5 nM. Meanwhile, the CTL activities toward Raji cells were not affected within a dasatinib-loaded MTLs of 1-10 nM. Dasatinib at 10 nM and above became inhibitory to T cells.

### EMBODIMENT 13 TRAFsomes activities on γδT cells, IL-2 stimulated LAK cells and aCD3/aCD28 bead expanded T cells

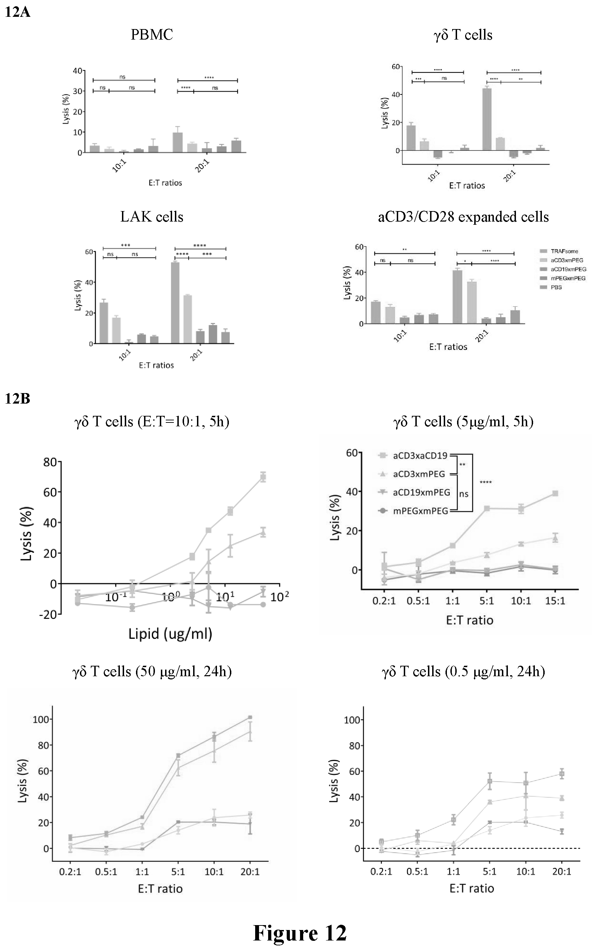

The MTLs were found to be able to stimulate other T cell subsets, including γδ T cells, IL-2-stimulated LAK cells or anti-CD3/anti-CD28 antibodies (aCD3/aCD28) expanded T cells. Compared to freshly isolated PBMCs, these pre-conditioned T cells were considered more reactive. Indeed, treatments with MTLs led to substantial cytolytic activity by γδ T cells (44.3% when E:T = 20:1) within 5 hours (hr) of incubation (FIGs. 12A-12B). Data shown in FIG. 12B were means ± SEM (n = 3). *p < .05; **p <.01; ***p < .001; ****p < .0001 by Student's t test. Similarly, MTLs trigger much higher CTL activities by IL-2 pre-stimulation LAK cells, compared to mPEG liposomes as well as anti-CD3 liposomes, especially with higher E:T ratios (FIG. 12A, center right). Similar activities were seen using aCD3/aCD28 expanded T cells, but the improvement by MTLs over anti-CD3 Fab' only liposomes were small (FIG. 12A, right). The MTLs took longer to activate resting T cells derived from PBMC than pre-stimulated T cell subsets. Without being bound by theory, the change in activation time may be due to the lack of TCR clustering in resting T cells and indicates that MTLs may be segregating TCR due to simultaneous cancer cell binding.

The above are only some preferred embodiments of the present disclosure instead of limitations on the present disclosure in any form or substance. It should be noted that, for those skilled in the art, improvements and supplements may be made without departing from the method of the present disclosure, the improvements and supplements shall also be covered by the protection of the present disclosure. The equivalent changes of alternations, modifications and evolutions can be made by those skilled in the art using the technical contents revealed above and without departing from the spirit and scope of the present disclosure, and those equivalent changes are regarded as equivalent embodiments of the present disclosure. Meanwhile, any alterations, modifications, and evolutions of any equivalent changes made to the above embodiments according to the essential technology of the present disclosure still fall within the scope of the technical solution of the present disclosure.

## Claims

1. A T cell redirection immunoliposome, comprising a liposome, and a targeting antibody, or a target-binding fragment thereof, and an immune cells binding antibody or a target-binding fragment thereof, modified on a surface of the liposome, wherein the targeting antibody, or the target-binding fragment thereof specifically binds to a target molecule on a surface of a target cell, and the immune cells binding antibody or the target-binding fragment thereof specifically binds to an immune effector cell.

2. The T cell redirection immunoliposome according to claim 1, wherein the targeting antibody or target-binding fragment thereof, is an anti-Her2/neu antibody or target-binding fragment thereof, and the immune cells binding antibody or the fragment thereof is an anti-CD3 antibody or target-binding fragment thereof.

3. The T cell redirection immunoliposome according to claim 2, wherein the average valency of the targeting antibody or the binding fragment thereof per liposome is 10, the valency is calculated by dividing the molar concentration of the contained antibodies or fragments thereof by the number of moles of liposomes.

4. The T cell redirection immunoliposome according to claim 2, wherein the average valency of the immune cells binding antibody or the fragment thereof per liposome is 6.

5. The T cell redirection immunoliposome according to claim 2, wherein the liposome contains an anti-cancer drug, an immunomodulator drug, or a combination of an anti-cancer drug and an immunomodulator drug.

6. The T cell redirection immunoliposomee according to claim 2, wherein the immunomodulator drug comprises resiquimod, imiquimod, Selgantolimod, Vidutolimod or combinations of two or more thereof.

7. The T cell redirection immunoliposome according to claim 2, wherein the immunomodulator drug is resiquimod.

8. The T cell redirection immunoliposome according to any one of claims 1-7, wherein components of the liposome comprise phosphatidylcholine, cholesterol, a lipid linked to the targeting antibody or target-binding fragment thereof, and a lipid molecule linked to the immune cells binding antibody or the fragment thereof.
